# EUROPEAN PATENT APPLICATION

(11) **EP 2 546 200 A1**
(43) Date of publication of application: **16.01.2013**
(21) Application number: 12175559.9
(22) Date of filing: 09.07.2012
(51) Int. Cl.: C02F 1/32, C02F 1/72, B23Q 11/10, A61L 2/10, B01D 17/04, C02F 101/32

(54) **Method and device for treating fluids**

(30) Priority: 11.07.2011 SE 1150658
(71) Applicant: Wallenius Water Aktiebolag, 112 51 Stockholm (SE)
(72) Inventor: Ålander, Eva, 181 34 Lidingö (SE)
(74) Representative: Bergström, Johan Erik

(57) **Abstract**

Method for treating fluids containing amounts of tramp oil comprising the steps of placing a treatment unit (20, 20') inside a volume of fluid to be treated, which treatment unit (20, 20') comprises a UV radiation member capable of emitting. UV radiation, radiating said volume of fluid with UV radiation, whereby said UV radiation is capable of creating radicals in said fluid, which radicals react with said tramp oil for causing it to polymerise and coagulate, rendering it easily separated from the cutting fluid.

## Description

### TECHNICAL FIELD

The present invention relates to a method and device for treating fluids and in particular a device for treating cutting fluids.

### BACKGROUND OF THE INVENTION

There are a number of methods and devices on the market that are capable of treating, and in particular purifying, liquids. In many instances, members capable of generating UV radiation are used in the treatment process, and in particular UV radiation of certain wave-lengths that can create different reaction components such as ozone, hydrogen peroxide, radicals, to mention a few. In order to increase the performance of the cleaning process, some treatment devices are arranged with catalysts that are capable of creating e.g. photo-catalytic reactions for producing the treatment components.

The above mentioned treatment methods have a good performance when treating liquids that possess a good transmissibility, i.e. the radiation from the UV generating members can spread in the volume to be treated. The more the liquid becomes filled with particles and the like matter increasing the opacity of the liquid, the more difficult it becomes to reach all parts of the volume of liquid to be treated.

One area where treatment and purification of liquid is important is machining where cutting fluids or coolants are used, Cutting fluids often comprise an emulsion of oil and water, where the water makes them susceptible to bacteria and other micro-organisms, leading to odours, and also leading to breaking down of the function of the cutting fluid and clogging of equipment and piping handling the cutting fluid, Further the cutting fluids become increasingly loaded with foreign matter from the machining during use.

In order to solve the problem, a few attempts have been made to purify cutting fluids with technologies involving no chemicals, where some are attempting the use of UV radiation. The applicant of the present invention has developed a method for treating cutting fluids involving no chemicals. Instead the cutting fluid is treated with UV radiation members that radiate a volume of cutting fluid, wherein the UV radiation is capable of creating radicals in the cutting fluid, where the radicals react with matter in the fluid, thereby treating it.

A common type of contamination in cutting fluids is tramp oil, also known as sump oil, which is unwanted oil that has mixed with cutting fluid. It originates as lubrication oil that seeps out from the slideways and washes into the coolant mixture, as the protective film with which a steel supplier coats bar stock to prevent rusting, or as hydraulic oil leaks. In extreme cases it can be seen as a film or skin on the surface of the coolant or as floating drops of oil.

The tramp oil has a tendency to promote the growth of bacteria and other micro-organisms in the cutting fluid, functioning as a nutrient. This is very unfavourable for the cutting fluid and makes the micro organism control much more difficult. Also the handling/removal of the oil entails additional equipment. Skimmers are sometimes used to separate the tramp oil from the coolant. These are typically slowly rotating vertical discs that are partially submerged below the coolant level in the main reservoir. As the disc rotates the tramp oil clings to each side of the disc to be scraped off by two wipers, before the disc passes back through the coolant. The wipers are in the form a channel that then redirects the tramp oil to a container where it is collected for disposal.

There is thus a need for a treatment method and device that can handle the above aspects of treatment of cutting fluids.

### BRIEF DESCRIPTION OF THE INVENTION

The aim of the present invention is to provide a method and device for treating cutting fluids where any oil residues such as tramp oil in coolants can be handled such that growth of bacteria and micro-organisms in the cutting fluid are prevented.

This aim is obtained by a method according to the independent patent claims. Preferable embodiments form the subject of the dependent claims.

According to a man aspect of the invention it is characterised in a method for treating fluids containing amounts of tramp oil comprising the steps of placing a treatment unit inside a volume of fluid to be treated, which treatment unit comprises a UV radiation member capable of emitting UV radiation, radiating said volume of fluid with UV radiation, whereby said UV radiation is capable of creating radicals in said fluid, which radicals react with said tramp oil for causing it to polymerise.

According to another aspect of the invention, said UV radiation is capable of creating photo-ionization effects in the fluid.

According to a further aspect of the invention, said treatment unit further comprises a quartz glass of high purity positioned between said UV radiation member and said fluid to be treated.

According to yet an aspect of the invention, it further comprises the step of creating a flow in said fluid to be treated.

There are a number of advantages with the present invention. The tramp oil is effectively removed by conventional separation after polymerization and coagulation achieved by the treatment unit. This effectively prevents any tramp oil from adversely affecting the liquid and preventing any growth of bacteria and other micro-organisms.

Thus the method according to the present invention both treats the liquid as such removing any bacteria and other micro-organisms directly as well as removing and/or destroying any tramp oil, without using any addition of chemicals and the like.

These and other aspects of and advantages with the present invention will become apparent from the following detailed description and from the accompanying drawings.

### BRIEF DESCRIPTION OF THE DRAWINGS

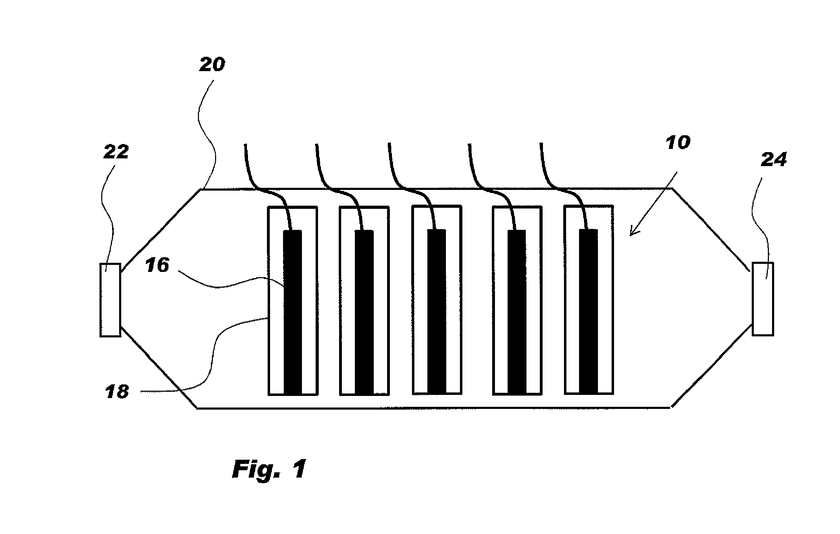

In the following detailed description of the invention, reference will be made to the accompanying drawings, of which
- Fig. 1: is a schematic side view of a treatment unit used in the present invention,
- Fig. 2: is a schematic side view of an environment where the present invention may be utilized, and
- Fig. 3: is a schematic side view of another embodiment of the present invention.

### DETAILED DESCRIPTION OF THE INVENTION

The present invention relates on the use of a treatment unit 10 comprises at least one radiation source 16 capable of creating radiation energy in the liquid. The radiation source is preferably capable of creating radiation in the UV region, due to the positive effects that UV radiation possess.

In order to create a good treatment environment and in order to maximize the treatment efficiency of the UV radiation, different measures are provided. One such measure for the treatment unit 10 is to position a transparent glass cover 18 or wall between the UV radiation source 16 and the fluid 14 to be treated. In order to create a desired photo-ionisation effect, which is very effective in treating the liquid, the glass cover 18 is made of very pure quartz glass. Preferred wavelengths are in the region of 100 nm to 220 nm, with preferable peaks between 170-190 nm. These highly energetic wavelengths are absorbed by water molecules, causing homolysis of the water molecules with the formation of hydrogen atoms and hydroxyl radicals.

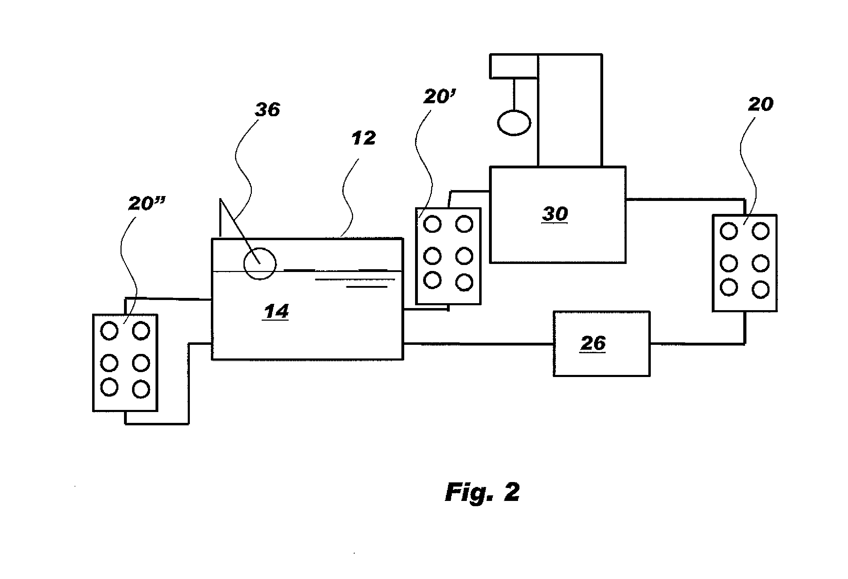

The process will create radicals having highly oxidizing effects, which are used for breaking down and decomposing any organic or biological material in the fluid. According to one aspect of the invention, the treatment unit, or preferably several treatment units 10, are placed in an enclosure or reactor 20, having an inlet 22 and an outlet 24 for the liquid to be treated to flow through the enclosure. The treatment units 10 are then positioned such in the enclosure that preferably the total volume of liquid is exposed to UV radiation in order that the photo-ionisation occurs throughout the volume. According to Fig. 2, the treatment reactor 20 can be placed in a normal loop for the liquid between a storage tank 12 and a cutting machine 30 such as a lathe, a miller, and the like, either before 20 or after 20' the cutting machine. It is also feasible to only have a separate loop with the reactor 20" connected to the tank 12 where the liquid is circulated and treated in the reactor. Further, it might be advantageous to position a filter 26, such as a band filter or the like, after the treatment reactor 20, which is capable of removing any coagulated tramp oil after the treatment in the treatment reactor 20.

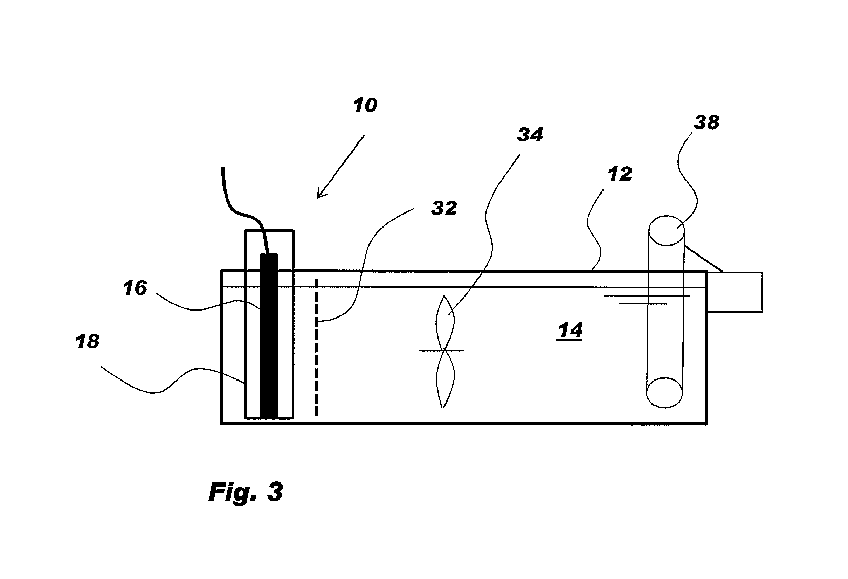

As an alternative, one or several treatment units can be positioned directly in the tank 12 containing fluid 14 to be treated, Fig. 3. The volume 12 could e.g. be a compartment which is common in a cutting machine, where cutting fluid is used during machining for cooling and lubricating the machining process.

As an extra feature, also creation of radicals by photo-catalytic processes is very effective for treating fluids. Hereby catalytic material is placed in the vicinity of the UV radiation sources such that the material is radiated, Fig. 3. The presence of photo-catalytic material increases or creates decomposing radicals. The photo-catalytic material could be placed on suitable carriers 32 such as metal plates, meshes or even attached to the surface of the surrounding glass. Suitable photo-catalytic materials include noble metals, Ti02, Si02, just to mention some.

When a number of such units are placed in the volume of liquid to be treated, a thorough treatment effect is obtained throughout the volume, cleaning the fluid and removing any organic material. Additional functions may include stirrers 34, and/or baffles that create and control flows in the fluid to be treated, either in the reactor or in the tank containing the liquid.

According to a certain aspect the above mentioned treatment device produces hydroxyl radicals that are formed in the treatment process, which hydroxyl radicals attack organic molecules by radical addition to the organic compound (unsaturated or aromatic) that contain a err bond (1), by hydrogen abstraction of a saturated aliphatic compound (2), and by electron transfer in the formation of ions of a higher valance (3).

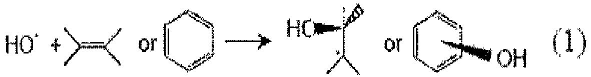

HO* + R - H → R* + H₂O (2)

HO* + R - X → [R-X]⁺* + HO⁻ (3)

Both hydrogen abstraction and radical addition produce reactive organic radicals. The organic radicals undergo subsequent oxidation forming carboxylic acids and hydro-peroxides.

At oxidation of lubricating oils the oxygenated by-products, such as carboxylic acids and hydro-peroxides, are known to combine to form larger molecular species. When a number of such molecules combine, the process is called polymerization, it results in the formation of large molecules of high molecular weight. Allowed to progress, polymerization continues to such an extent that a sticky sludge of high viscosity is formed. In hydraulic systems this material is problematic causing clogging and fouling. However, in coolant systems with applied treatment according to the invention, the oxidation polymerization of tramp oil allows for separation of the unwanted tramp oil as coagulated sludge e.g. in a filtration system.

It is thus easy to collect and remove the coagulated tramp oil from the surface of the treatment vessel or storage tank 12. This collection and removal may be performed by a number of different methods and devices. One is for example to use a scraper or the like member 36, Fig. 2, that is moved along the surface of the liquid, which scraper removes any polymerized and coagulated oil due to the treatment. Another example is shown in Fig. 3, where a conventional belt skimmer 38 is used, having an endless belt arranged vertical in the tank where the upwards moving part of the belt brings the coagulated oil with it from the surface of the liquid.

It is to be understood that the embodiments described above and shown in the drawings only are to be regarded as non-limiting examples of the invention and that it may be modified in many ways within the scope of the patent claims.

## Claims

1. Method for treating fluids containing amounts of tramp oil comprising the steps of
- placing a treatment unit inside a volume of fluid to be treated, which treatment unit comprises a UV radiation member capable of emitting UV radiation,
- radiating said volume of fluid with UV radiation, whereby said UV radiation is capable of creating radicals in said fluid, which radicals react with said tramp oil for causing it to polymerise and coagulate, rendering it easily separated from the cutting fluid.

2. Method according to claim 1, wherein said UV radiation is capable of creating photo-ionization effects in the fluid.

3. Method according to claim 2, wherein said treatment unit further comprises a quartz glass of high purity positioned between said UV radiation member and said fluid to be treated.

4. Method according to any of the preceding claims, further comprising the step of creating a flow in said fluid to be treated.

5. Device for performing the steps of any of the claims 1 to 6.

6. Device according to claim 5, wherein it further comprises a collection mechanism for collecting said polymerised tramp oil.
